## Europäisches Patentamt

## European Patent Office

## Office européen des brevets

(19)

(11) Veröffentlichungsnummer: **0 075 762**
**B2**

(12) **NEUE EUROPÄISCHE PATENTSCHRIFT**

(45) Veröffentlichungstag der neuen Patentschrift:
**14.06.89**

(51) Int. Cl.⁴: **A 61 M 5/14**

(21) Anmeldenummer: **82108368.0**

(22) Anmeldetag: **10.09.82**

(54) Zur Implantation in einen lebenden Körper vorgesehenes Infusionsgerät.

(30) Priorität: **25.09.81 DE 3138320**

(43) Veröffentlichungstag der Anmeldung:
**06.04.83 Patentblatt 83/14**

(45) Bekanntmachung des Hinweises auf die Patenterteilung:
**09.04.86 Patentblatt 86/15**

(45) Bekanntmachung des Hinweises auf die Entscheidung
über den Einspruch:
**14.06.89 Patentblatt 89/24**

(84) Benannte Vertragsstaaten:
**AT CH DE FR GB IT LI**

(56) Entgegenhaltungen:
**EP-A- 0 019 817**
**DE-A- 1 918 684**
**DE-A- 2 920 975**
**US-A- 3 527 220**
**US-A- 3 647 117**
**US-A- 3 731 681**
**US-A- 3 765 414**
**US-A- 4 013 074**
**US-A- 4 190 426**

(73) Patentinhaber: **Siemens Aktiengesellschaft Berlin und München, Wittelsbacherplatz 2, D-8000 München 2 (DE)**

(72) Erfinder: **Franetzki, Manfred, Dr., Schleifweg 7b, D-8521 Usttenreuth (DE)**
Erfinder: **Prestele, Karl, Bismarckstrasse 21 d, D-8520 Erlangen (DE)**

EP 0 075 762 B2

## Beschreibung

Zur Implantation in einem lebenden Körper vorgesehenes Infusionsgerät.

Die Erfindung betrifft ein implantierbares Medikament-Infusionsgerät nach dem ersten Teil von Anspruch 1.

Ein Infusionsgerät der eingangs genannten Art ist beispielsweise aus der DE-A 2 721 752 bekannt. Dort ist ein Gerät für die Zuführung von Medikamenten zum menschlichen oder tierischen Körper beschrieben, bei dem in der Gehäusewand eine Membran eingelassen ist. Die Membran soll speziell hydrophile Eigenschaften haben, um mittels einer Förder- und Dosiereinheit Körperflüssigkeit durch die Membran anzusaugen, um darin ein festes Medikament lösen zu können. In dieser Druckschrift wird auf Probleme, die durch einen ungewollten Überdruck im Vorratsbehälter auftreten können, nicht eingegangen. Diese Probleme sind für das bekannte Gerät aufgrund des festen Medikamentenblocks und der dort gewählten konstruktiven Anordnung der Förder- und Dosiereinheit auch nicht von Bedeutung.

Aus der DE-A 2 920 975 ist ein extrakorporales Medikamenten-Infusionsgerät mit einer gasdurchlässigen, aber flüssigkeitsundurchlässigen Membran bekannt. Dabei ist jedoch kein Druckausgleich zwischen einer Flüssigkeit innerhalb des Gerätegehäuses und den Körpersubstanzen ausserhalb des Gerätegehäuses vorgesehen.

In der US-A 4 190 426 ist ein Gasabscheidefilter beschrieben, das zum Abscheiden von Gas aus Flüssigkeiten dient, die Patienten verabreicht werden. Dabei wird durch eine gasdurchlässige Membran ein bereits aus der Infusionsflüssigkeit abgeschiedenes Gas nach aussen abgeführt.

In der US-A 4 013 074 ist ein implantierbares Medikamentendosiergerät offenbart, in dessen Gehäuse ein erstes Reservoir für die Infusionslösung und ein zweites Reservoir als Pufferspeicher für entnommene Infusionslösung vorgesehen sind. Jedes der beiden Reservoire ist durch eine Öffnung mittels einer Spritze von aussen zugänglich. Auf einen permanent vorhandenen Druckausgleich zwischen Innenraum und dem Aussenraum des implantierten Gehäuses wird in dieser Druckschrift nicht eingegangen.

Schliesslich ist aus der DE-A 2 652 026 ein Infusionsgerät bekannt, bei dem aus Sicherheitsgründen vorgesehen ist, die Infusionsflüssigkeit im Vorratsbehälter auf einem Druck zu halten, der niedriger ist als der Druck an der Ausflussöffnung des Katheters. Durch eine solche Massnahme wird verhindert, dass sich durch ein Leck von Kapsel oder Pumpe unbeabsichtigt Flüssigkeit aus dem Vorratsbehälter in den Patientenkörper ergiessen kann. Zur Durchführung dieser Massnahme wird beispielsweise ein flexibler Flüssigkeitsbehälter von einer Substanz beaufschlagt, die bei Körpertemperatur einen entsprechenden Dampfdruck hat. Es ist auch möglich, in das dicht gekapselte Gerätegehäuse ein Puffergas mit einem solchen Dampfdruck einzufüllen, so dass auch bei vollem Flüssigkeitsvorratsbehälter noch ein Unterdruck im Vergleich zum Druck der Umgebung erhalten bleibt. Eine hermetische Kapselung des Gerätegehäuses mit Aufrechterhaltung von Unterdruck über längere Zeiten bringt jedoch auch eine Reihe von Problemen mit sich. Beispielsweise müssen Temperaturschwankungen und äussere Luftdruckänderungen, welche die Druckdifferenz zwischen dem Körpergewebe und dem Gehäuseinneren verändern können, berücksichtigt werden. Dadurch muss von vergleichsweise grossem Unterdruck, beispielsweise bis zu 0,5 bar, ausgegangen werden. Dies hat jedoch zur Folge, dass speziell bei Verwendung von Rollenpumpen als Förder- und Dosiereinheit die Gefahr des Rückflusses von Flüssigkeit im Katheter während des Abhebens der Rollen besteht. Bei dem bekannten implantierbaren Medikamenten-Infusionsgerät ist somit der Medikamentenspeicher mit einem Unterdruck beaufschlagt. Es ist keine gasdurchlässige Membran zum Druckausgleich zwischen der Flüssigkeit innerhalb des Gerätegehäuses und den Körpersubstanzen ausserhalb des Gerätegehäuses vorgesehen.

Aufgabe der Erfindung ist es, ein Infusionsgerät der eingangs genannten Art so auszugestalten, dass relativ kurzzeitig auftretende Überdrucke den Patienten nicht gefährden.

Diese Aufgabe wird erfindungsgemäss dadurch gelöst, dass die von der Förder- und Dosiereinheit geförderte Flüssigkeit das in flüssiger Form im Vorratsbehälter vorliegende Medikament ist und dass die Membran gasdurchlässig, aber flüssigkeitsundurchlässig ist.

Der besondere Vorteil der Erfindung besteht darin, dass der Druckausgleich zwischen der Flüssigkeit innerhalb des Gerätegehäuses und den Körpersubstanzen ausserhalb des Gerätegehäuses selbsttätig erfolgt, so dass es keines äusseren, den Patienten zusätzlich belastenden Eingriffes bedarf, um einen Druckausgleich zur Vermeidung kritischer Situationen herbeizuführen.

Nach einer Weiterbildung der Erfindung gemäss Patentanspruch 2 kann die Membran von Fall zu Fall über einen Schlauch an eine solche Stelle des Körpers verlegt werden, die für einen Druckausgleich durch Gasaustausch besonders geeignet ist.

In einer weiteren Ausführung der Erfindung nach Patentanspruch 3 können die Poren der Membran so bemessen sein, dass unter Inkaufnahme einer längeren Periode für den Druckausgleich die Poren so klein bemessen sind, dass keine Bakterien oder Körperzellen in das Gehäuseinnere eintreten können.

Durch die im Patentanspruch 4 beschriebene zusätzliche Massnahme ist erreicht, dass auch bei sehr kurzfristig auftretenden Druckunterschieden, beispielsweise beim Nachfüllen des Medikamentenbehälters, ein schneller Druckausgleich mit der Körperumgebung stattfinden kann, ohne den Patienten durch eine zusätzliche Einstichstelle, die nur dem kurzfristigen Druckausgleich dient, zu belasten.

Einzelheiten der Erfindung ergeben sich aus der nachfolgenden Figurenbeschreibung von Ausführungsbeispielen anhand von Zeichnungen. Es zeigen jeweils in **schematischer Darstellung** die

Fig. 1 bis 4 unterschiedlich aufgebaute Infusionsgeräte im Querschnitt und

Fig. 5 ein Infusionsgerät in Aussenansicht.

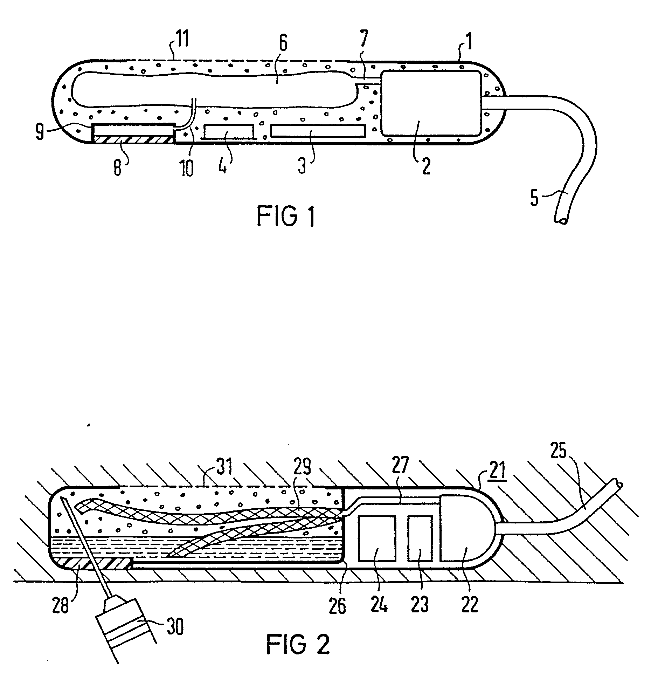

In der Fig. 1 bedeutet 1 ein Gerätegehäuse eines implantierfähigen Infusionsgerätes. Dieses hat als flache Kapsel in etwa eine ähnliche Raumform wie das Gehäuse eines Herzschrittmachers, und es besteht aus implantierfähigem und mit Körpergewebe verträglichem Material, beispielsweise Titan. Im Gerätegehäuse 1 sind eine Förder- und Dosiereinheit 2 sowie eine Einheit 3 mit elektronischen Schaltkreisen für Antrieb und Steuerung der Förder- und Dosiereinheit 2 sowie eine Energieversorgungseinheit 4 (Batterie) angedeutet; 5 kennzeichnet einen Katheter, der innerhalb des Patientenkörpers an eine geeignete Stelle gelegt werden kann. Mit 6 ist ein Vorratsbehälter für eine Infusionsflüssigkeit bezeichnet.

Über ein im Gerätegehäuse eingebrachtes Nachfüllseptum 8 mit Verbindungsleitung 10 kann beispielsweise Insulin als Infusionsflüssigkeit perkutan in den Vorratsbehälter 6 eingebracht werden. Zu diesem Zweck besteht das Nachfüllseptum 8 aus einem selbstschliessenden Elastomer, das mit einer üblichen Injektionskanüle durchstochen werden kann.

Der Vorratsbehälter 6 für die Flüssigkeit ist durch einen sogenannten Nadelstopper 9 gegen Beschädigung beim Nachfüllen von Infusionsflüssigkeit geschützt. Innerhalb des Vorratsbehälters 6 kann sich ein Förderdocht befinden, von dem mittels der Förder- und Dosiereinheit 2 die Flüssigkeit über einen Förderschlauch 7 blasenfrei zum Ausflusskatheter 5 abgesaugt wird.

Soweit ist der Aufbau eines Infusionsgerätes vom Stand der Technik bekannt. Während die bekannten Infusionsgeräte zwecks Aufrechterhaltung von Unterdruck im Gerätegehäuse hermetisch gekapselt sind, ist nun in die Wand des Gerätegehäuses 1 eine Membran 11 eingelassen.

Bei der Ausführungsform nach Fig. 1 besteht die Membran 11 aus hydrophobem Material mit so feinen Poren, dass auch bei maximalem Differenzdruck zwischen dem Gehäuseinneren und der Umgebung keine Körperflüssigkeit durch die Poren hindurchgelassen wird. Gleiches gilt für Körperzellen und/oder Bakterien. Hierzu können übliche hydrophobe Filter verwendet werden, die beispielsweise aus Polyolefinen, PTFE oder ähnlichen Materialien bestehen.

Bei einer solchen Ausbildung des Infusionsgerätes ist sichergestellt, dass bei kontinuierlicher Flüssigkeitsabgabe aus dem Vorratsbehälter das im umgebenden Körpergewebe oder in der Körperflüssigkeit gelöste Gas in das Gerätegehäuse eintreten kann. Damit ist für weitgehenden Druckausgleich gesorgt. Da der Gasdruck im Körpergewebe bzw. der Körperflüssigkeit näherungsweise dem äusseren Luftdruck entspricht, ist auch eine Anpassung an den barometrischen Druck der Umgebung gewährleistet.

Beim transkutanen Nachfüllen des Vorratsbehälters ist es zweckmässig, stets den Restinhalt von Flüssigkeit im Vorratsbehälter zunächst abzusaugen. Bei einem solchen vergleichsweise schnellen Vorgang kann praktisch kein Gas aus der Umgebung des Gerätegehäuses nachdiffundieren, so dass kurzzeitig ein Unterdruck entstehen kann. Beim unmittelbar anschliessenden Nachfüllen des Vorratsbehälters wird das im Gerätegehäuse vorhandene Gas in das umliegende Körpergewebe verdrängt und dort absorbiert. Es kann zweckmässig sein, eine evtl. entstehende geräteexterne Gasblase abzusaugen.

In der Fig. 2 entsprechen die Bezugszeichen 21 bis 28 sowie 31 in ihrer Durchnumerierung den um 20 erhöhten Bezugszeichen der Fig. 1. Speziell mit 29 ist ein Förderdocht im Vorratsbehälter bezeichnet. Bei der Ausführungsform der Fig. 2 ist der Vorratsbehälter 26 für die Infusionsflüssigkeit starr ausgebildet. Dies hat den Vorteil, dass ein Teil des Vorratsbehälters 26 gleichzeitig die Gehäusewand bildet und dass das Nachfüllseptum 28 unmittelbar in den Vorratsbehälter 26 führen kann. Dabei ist der Nadelstopper vorteilhafterweise direkt in die Gehäusewand integriert. Es ergeben sich also gewisse konstruktive Vereinfachungen. Mit 30 ist eine Spritze zum perkutanen Absaugen bzw. Nachfüllen der Infusionsflüssigkeit durch das angedeutete Körpergewebe bezeichnet.

Bei diesem Ausführungsbeispiel schliesst die Membran unmittelbar den Vorratsbehälter 26 mit Infusionsflüssigkeit vom Körpergewebe ab. Es dürfen keine Undichtigkeiten für Flüssigkeiten vorhanden sein, so dass die Hydrophie der Membran unabdingbare Voraussetzung ist.

Der Druckausgleich erfolgt bei dieser Ausführungsform der Erfindung durch unmittelbaren Gasaustausch zwischen Umgebung und Vorratsbehälter 26. Aus diesem Grunde ist mittels des Förderdochtes 29 das blasenfreie Absaugen von Flüssigkeit durch die Förder- und Dosiereinheit 22 sicherzustellen. Weiterhin muss auch dafür gesorgt sein, dass bei jedem Füllungszustand des Vorratsbehälters 26 und jeder möglichen Patientenlage der Förderdocht 29 mit Infusionsflüssigkeit in Kontakt ist.

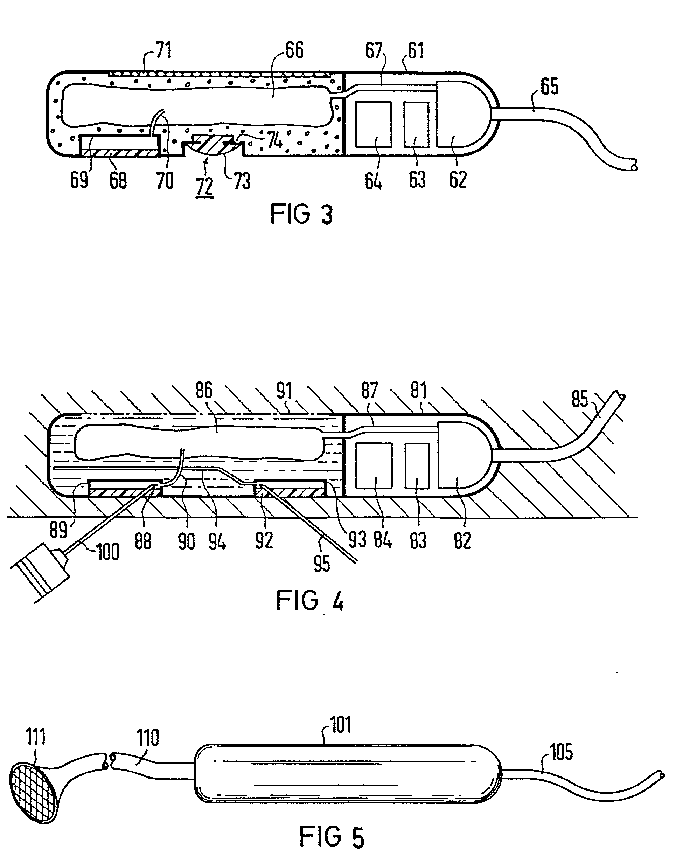

Das Ausführungsbeispiel gemäss Fig. 3 ist prinzipiell entsprechend Fig. 1 aufgebaut, wobei sich entsprechende Einheiten in der Durchnumerierung von 61 bis 71 ergeben. Als Membran ist hier speziell eine sogenannte Diffusions-Membran 71 mit hoher Permeabilität für Gase vorgesehen, die beispielsweise aus Silikon besteht. Der Druckausgleich zwischen dem Gehäuseinneren und Umgebung erfolgt also bei dieser Ausführungsform der Erfindung allein durch Diffusion, was bei der üblicherweise langsamen Entleerung des Vorratsbehälters aufgrund der Mikrodosierung durch die Förder- und Dosiereinheit 62 vollständig genügt. Da beim Nachfüllen aber eine diffusionsbestimmte Geschwindigkeit des Gasaustausches nicht ausreicht, ist zusätzlich wiederum ein Ventil 72 vorhanden. Dadurch ist beim perkutanen Nachfüllen des Vorratsbehälters 66 ein gegenüber der Diffusionsgeschwindigkeit wesentlich schnelleres Entweichen des Gases aus dem Gerätegehäuse 61 möglich. Als Alternative zu einem Ventil ist es auch möglich, dass eine solche elastische Diffusions-Membran verwendet wird, die sich nach aussen auswölbt, bis der Überdruck durch Diffusion abgebaut ist.

Das Ventil 72 kann aus einem in die Wand des Gerätegehäuses 61 eingelassenen Gummipfropfen 73 bestehen, der eine Öffnung 74 in der Gehäusewandung abdeckt und im Normalfall dicht verschliesst, bei Überdruck im Gehäuseinneren dagegen öffnet.

In der Fig. 4 bedeuten die Bezugszeichen 81 bis 91 die anhand der vorangehenden Figuren beschriebenen Einheiten in entsprechender Durchnumerierung. Zusätzlich zum Flüssigkeitsnachfüllseptum 88 ist ein weiteres Septum 92 als separates Einstichsystem vorhanden, das wiederum aus selbstschliessendem Elastomer mit anschliessendem Nadelstopper 93 besteht. Vom Septum 92 ist ein interner Schlauch 94 zur tiefsten Gehäusestelle geführt.

Beim Infusionsgerät nach der Fig. 4 erfolgt der Druckausgleich beim langsamen Fördervorgang und Abpumpen des Vorratsbehälters 86 durch die Förder- und Dosiereinheit 82 entsprechend den vorher beschriebenen Ausführungsbeispielen über die gasdurchlässige Membran 91. Der Druck- bzw. Volumenausgleich beim perkutanen Nachfüllen des Vorratsbehälters erfolgt nun aber nicht durch Gasaustausch mit dem umgebenden Körpergewebe, sondern mit der Umgebung ausserhalb des Patientenkörpers. Ein solcher Ausgleich kann besonders schnell durchgeführt werden; gleichzeitig werden die im Gerätegehäuse vorübergehend angesammelten Substanzen entfernt.

Für den Nachfüllvorgang wird also in das zweite Septum 92 eine Kanüle 95 eingestochen, die durch das Gewebe nach aussen führt. Dieses zweite Septum 92 wird zweckmässigerweise so angeordnet, dass unmittelbar mit der Einstichkanüle aus den tiefliegenden Gehäuseteilen Kondenswasser oder andere gegebenenfalls eingedrungene Körperflüssigkeiten abgesaugt werden können.

Der bei der Fig. 4 vorhandene weitere Zugang zum Kapselinneren hat daneben auch Vorteile bei der Herstellung und Kapselung des gesamten Infusionsgerätes.

Bei den Infusionsgeräten nach Fig. 1 sowie 3 und 4 ist im Gerätegehäuse jeweils ein flexibler Vorratsbehälter für die Infusionsflüssigkeit vorgesehen. Die Flüssigkeit wird im Normalfall mittels einer Kanüle bzw. Spritze durch das Septum blasenfrei eingefüllt. Allerdings kann es auch bei diesen Ausführungsformen nützlich sein, einen zusätzlichen Absaugdocht entsprechend Fig. 2 vorzusehen, um mit Sicherheit auch bei evtl. vorhandenen Gasblasen ein blasenfreies Abpumpen der Flüssigkeit zu gewährleisten.

Bei den vorangegangenen Figuren wurde die jeweils unterschiedlich ausgebildete Membran 11, 31, 71 und 91 immer als Teil der Gehäusewandung dargestellt. Es kann aber zweckmässig sein, eine solche Membran über einen zusätzlichen Schlauch mit dem Gerätegehäuse zu verbinden.

In der Fig. 5 ist mit 101 ein entsprechend ausgebildetes Gerätegehäuse mit Katheter 105 sowie einem Schlauch 110 mit abschliessender Membran 111 gekennzeichnet. Der Schlauch 110 mündet dabei rückseitig in der entsprechenden Kammer des Gerätegehäuses 101 und ist so lang gewählt, dass er im Patientenkörper bis zu einem Reservoir von Körperflüssigkeit legbar ist. Auch der Schlauch 110 selbst kann aus Membranmaterial bestehen.

Die anhand der verschiedenen Ausführungsformen beschriebenen erfindungsgemässen Infusionsgeräte können insbesondere als künstliches Pankreas zur Insulinverabreichung bei der Diabetes-Therapie verwendet werden. Dabei sind mit der Erfindung entscheidende Probleme bei der Implantation solcher Geräte in einen lebenden Körper überwunden. Der Druckausgleich zwischen Gehäuseinnenraum und Gehäuseumgebung kommt jeweils dadurch zustande, dass während des Betriebes des implantierten Infusionsgerätes die infundierte Flüssigkeit durch Körpergase ersetzt wird. Dabei umschliesst in den Fig. 1, 3 und 4 der flexible Vorratsbehälter jeweils die gesamte (nicht sichtbar dargestellte) Infusionsflüssigkeit, während in Fig. 2 der von der Infusionsflüssigkeit nicht ausgefüllte Raum in starren Vorratsbehälter durch Körpergase ausgefüllt wird.

Durch obige Massnahmen ist auch bei sich ändernden Raumlagen des Infusionsgerätes aufgrund von Lageveränderungen des Patientenkörpers unabhängig vom Füllstand des Vorratsbehälters immer ein sicheres Absaugen der Infusionsflüssigkeit gewährleistet. Die zusätzliche Anordnung eines Überdruckventiles in der Gehäusewand dient insbesondere zum Ausgleich relativ kurzfristig auftretender Überdrucke, z.B. im Flugzeug oder beim Auffüllen des Vorratsbehälters.

**Patentansprüche**

1. Implantierbares Medikamenten-Infusionsgerät mit einem Gerätegehäuse (1, 21, 61, 81, 101), mit einem im Gerätegehäuse untergebrachten Vorratsbehälter (6, 26, 66, 86) für das Medikament, mit einer Förder- und Dosiereinheit (2, 22, 62, 82) zum Fördern einer Flüssigkeit, die sich im Gerätegehäuse befindet, und mit einer Membran (11, 31, 71, 91) als Druckausgleichsvorrichtung zum Druckausgleich zwischen der Flüssigkeit innerhalb des Gerätegehäuses und den Körpersubstanzen ausserhalb des Gerätegehäuses, dadurch gekennzeichnet, dass die von der Förder- und Dosiereinheit (2, 22, 62, 82) geförderte Flüssigkeit das in flüssiger Form im Vorratsbehälter (6, 26, 66, 86) vorliegende Medikament ist und dass die Membran (11, 31, 71, 91) gasdurchlässig, aber flüssigkeitsundurchlässig ist.

2. Infusionsgerät nach Anspruch 1, dadurch gekennzeichnet, dass die Membran (111) Teil eines Schlauches (110) ist, der im Patientenkörper an eine geeignete Stelle legbar ist.

3. Infusionsgerät nach Anspruch 1 oder 2, dadurch gekennzeichnet, dass die Poren der hydrophonen Membran (11, 31, 71, 91) so bemessen ist, dass bei maximalem Druckunterschied zwischen dem Innenraum des Gerätegehäuses (1, 21, 61, 81, 101) und dessen Umgebung im Patientenkörper keine Körperzellen und/oder Bakterien hindurchtreten können.

4. Infusionsgerät nach einem der vorhergehenden Ansprüche, dadurch gekennzeichnet, dass zum Druckausgleich während des Nachfüllvorganges das Gerätegehäuse (61) im Bereich des den Vorratsbehälter umgebenden Innenraumes mit einer weiteren, durch ein Überdruckventil (72) verschliessbaren Öffnung versehen ist.

## Revendications

1. Appareil implantable pour la perfusion de médicaments, comprenant: un boîtier de l'appareil (1, 21, 61, 81, 101) un réservoir de réserve en médicament (6, 26, 66, 86) logé dans le boîtier de l'appareil, une unité de transport et de dosage (2, 22, 62, 82) pour transporter un liquide qui se trouve dans le boîtier de l'appareil (1, 21, 61), et une membrane (11, 31, 71, 91) servant de dispositif d'égalisation des pressions, destiné à égaliser la pression entre le liquide contenu dans le boîtier de l'appareil et les substances de l'organisme à l'extérieur du boîtier de l'appareil, caractérisé en ce que le liquide transporté par l'unité de transport et de dosage (2, 22, 62, 82) est le médicament se présentant sous forme liquide dans le réservoir de réserve (6, 26, 66, 86) et en ce que la membrane (11, 31, 71, 91) est perméable aux gaz, mais imperméable aux liquides.

2. Appareil de perfusion suivant la revendication 1, caractérisé en ce que la membrane (111) est une partie d'un tuyau souple (110) qui peut être placé en un endroit convenable dans l'organisme du patient.

3. Appareil de perfusion suivant la revendication 1 ou 2, caractérisé en ce que les pores de la membrane hydrophobe (11, 31, 71, 91) ont des dimensions telles que, pour une différence de pression maximale entre l'intérieur du boîtier (1, 21, 61, 81, 101) et ce qui l'entoure, aucune cellule de l'organisme et/ou bactérie ne peut pénétrer dans l'organisme du patient.

4. Appareil de perfusion suivant l'une des revendications précédentes, caractérisé en ce que, pour égaliser la pression pendant le processus de remplissage, le boîtier de l'appareil (61) est muni, dans la région de l'espace intérieur entourant le réservoir de réserve, d'un autre orifice qui peut être fermé par une soupape de surpression (72).

## Claims

1. Implantable medicine infusion device having a device housing (1, 21, 61, 81, 101), with feed container (6, 26, 66, 86) for the medicine which is accommodated in the device housing, a conveying and dispensing unit (2, 22, 62, 82) which serves to convey a liquid contained in the device housing, and a membrane (11, 31, 71, 91) which serves as pressure equalising device for equalising the pressure between the liquid within the device housing and the body subtances outside the device housing, characterised in that the liquid which is conveyed by the conveying and dispensing unit (2, 22, 62, 82) is the medicine which is contained in liquid form in the feed container (6, 26, 66, 86) and that the membrane (11, 31, 71, 91) is permeable to gas but impermeable to liquid.

2. Infusion device according to claim 1, characterised in that the membrane (111) forms part of a tube (110) which can be placed in the body of the patient at a suitable position.

3. Infusion device according to claim 1 or 2, characterised in that the pores of the hydrophobic membrane (11, 31, 71, 91) are so dimensioned that, with a maximum pressure difference between the internal space of the device housing (1, 21, 61, 81, 101) and its environment in the patient's body, no body cells and/or bacteria can pass through.

4. Infusion apparatus according to one of the foregoing claims, characterised in that for equalising pressure during the refilling of the device housing (61), there is provided in the region of the internal space surrounding the feed container a further opening closeable by a pressure relief valve (72).

FIG 1

FIG 2

FIG 3

FIG 4

FIG 5